# EUROPEAN PATENT SPECIFICATION

(11) **EP 1 637 297 B1**
(45) Date of publication and mention of the grant of the patent: **04.01.2012**
(21) Application number: 03816548.6
(22) Date of filing: 21.08.2003
(51) Int. Cl.: B26F 1/08, A61F 13/15

(54) **CUTTING DEVICE AND CUTTING METHOD, AND EQUIPMENT AND METHOD FOR MANUFACTURING INTER-LABIA MAJORA PAD**
SCHNEIDVORRICHTUNG UND SCHNEIDVERFAHREN SOWIE GERÄT UND VERFAHREN ZUR HERSTELLUNG EINES LABIA MAJORA-ZWISCHENPADS
DISPOSITIF ET PROCEDE DE DECOUPE, EQUIPEMENT ET PROCEDE DE FABRICATION DE SERVIETTES HYGIENIQUES

(30) Priority: 31.03.2003 JP 2003097147
(43) Date of publication of application: 22.03.2006
(73) Proprietor: UNI-CHARM CORPORATION, Shikokuchuo-shi Ehime 799-0111 (JP)
(72) Inventor: Mizutani, Satoshi, Kanonji-shi Kagawa 769-1602 (JP); Hosokawa, Masashi, Kanonji-shi Kagawa 769-1602 (JP); Yamaki, Koichi, Kanonji-shi Kagawa 769-1602 (JP); Noda, Yuki, Kanonji-shi Kagawa 769-1602 (JP)
(74) Representative: Main, Malcolm Charles
(86) International application number: PCT/JP2003/010565
(87) International publication number: WO 2004/087383

(56) References cited:
- EP-A2- 0 595 243
- WO-A-01/02159
- WO-A1-01/17473
- GB-A- 890 682
- JP-A- 1 058 496
- JP-A- 4 360 796
- JP-A- 8 052 696
- JP-U- 60 048 994
- US-A- 5 762 596

## Description

### FIELD OF THE INVENTION

The present invention relates, for example, to a cutter, an apparatus for producing an interlabial pad comprising the cutter, a cutting method, and a method for producing the interlabial pad comprising the cutting method.

### RELATED ART

Conventionally, cutters for cutting continuous sheet base materials have been known (for example, see the Laid-Open Japanese Patent Publication HEI 8-52696). A cutter disclosed in the Laid-Open Japanese Patent Publication HEI 8-52696 comprises, for example, a die cut roller which has a blade and an anvil roller being spaced under the die cut roller. Suction holes are provided in the portion within the blade of the die cut roller.

With this cutter, sanitary products are cut out from a raw material sheet by the blade through supplying the raw material sheet between the rollers. Then, the cut-out sanitary product is conveyed while being kept on the surface of the die cut roller by being sucked with a suction device.

However, with the above-described cutter, a remainder of the raw material sheet after the sanitary product is cut out may interfere with the cut-out sanitary product and cause dislocation of the sanitary product.

International Patent Publication No. WO 01/02159 discloses a method and calender device for calendering an absorbent layer formed from fibre material by means of a dry forming technique.

UK Patent No. 890, 682 describes a method of producing an absorbent fibrous body suitable for use as an absorbent component in absorbent products.

Japanese Patent No. 4-360796 describes a punching device for punching a product from a sheet body. Following punching of the sheet body, the remainder of the sheet body is sent to a separator and frame parts on both ends of the sheet body are pulled. This document forms the basis for the preamble of claim 1.

European Patent No. 0,595,243 describes a punching apparatus which punches a sheet of paper into products and separates the products from scrap.

International Patent Publication No. WO 01/17473 describes a method and apparatus for separating a discrete element from a first substrate web.

### SUMMARY OF THE INVENTION

An object of the present invention is to provide a cutter which can prevent the cut-out members being dislocated.

Specifically, the present invention provides the following.
(1) A cutter for cutting out a member from a continuous sheet of base material, comprising: a conveying means for conveying the continuous sheet of base material along a continuing direction of the continuous sheet of base material; a cutting means, having a cutting blade and a driving mechanism for bringing the cutting blade into substantially a center portion in a width direction of the conveyed base material, for cutting out the member by pressing the cutting blade against the base material; a member discharging means for discharging the cut-out member; and a dividing means for dividing a base material remainder, which is a remainder of the base material after the member is cut out, into two parts along the continuing direction of the base material such that the remainder of the base material is separated from the member; characterised in that said dividing means comprises: a slit forming device having a blade which forms slits as perforation cutting plane lines along substantially a centre of the continuous sheet of base material in the continuing direction; and in that said cutter further comprises a base material remainder discharging mechanism for discharging each of the two parts of the base material remainder in directions away from each other; and an embossing means for embossing the continuous sheet of base material, wherein said embossing means embosses the continuous sheet of base material such that an embossing rate of side portions of the continuous sheet of base material is higher than an embossing rate of the centre portion of the continuous sheet of base material in the width direction of the continuous sheet of base material.
   Examples of the base material may be absorbent materials which can absorb liquid. Specifically, the absorbent material contains, for example, hydrophilic fibers such as pulp, super absorbent polymer (SAP), synthetic fibers or their mixture. For example, SAP having about 310 g/m² by a weight per unit, pulp having about 400 g/m² by a weight per unit, furthermore, pulp containing about 20 to 30 % synthetic fiber by a weight per unit.
   Further, the thickness of the base material may be uniform or ununiform.
   The cutting blade is an edge, and there is no limit to the number and the shape thereof. The cutting blade may be mounted on the surface of a block or a roller. Also, the cutting blade may be moved relatively in the direction along a surface of the base material when the cutting blade is pressed against the base material. Further, the cutting blade may cut out two members at the same time.
   Examples of the conveying means may be a roller, belt conveyer, and the like.
   Examples of the member discharging means and the base material remainder discharging mechanism may be free fall of the member or the base material remainder due to its own weight in addition to the roller, the belt conveyer and the like.
   The members are used as sanitary products, diapers, and other absorbent articles, and the shapes may be determined as appropriate.
   Like the base material, it is preferable that the base material remainder is in a continuous form so that the base material remainder can be easily discharged by simply pulling the base material remainder.
   Discharging the two divided base material remainders in directions away from each other may mean to discharge them in such directions that the distance between the two base material remainders after cutting out the members increases. Specifically, the two divided base material remainders may be discharged to be extended on both sides of the discharging direction of the member. At this time, the angle between the discharging directions of the two divided base material remainders and the discharging direction of the base material may be determined as appropriate. However, with a large angle, the member and the base material remainder can be easily separated by utilizing the resistance of the member and the base material remainder to deformation.
   According to the present invention, the member and the base material remainder can be surely separated so that the dislocations of the cut-out members can be prevented.
(2) The cutter described above, wherein the base material remainder discharging mechanism discharges each of the two parts of the base material remainder in a thickness direction of the member at a predetermined angle to a discharging direction of the member.
   The base material is in a sheet form so that it is difficult to be bent in the width direction. However, it can be easily bent in the thickness direction. Therefore, it is preferable to discharge the two divided base material remainders in the thickness direction at a predetermined angle to the discharging direction of the member.
(3) The cutter described above, wherein the base material remainder discharging mechanism discharges each of the two parts of the base material remainder in substantially the same direction as a discharging direction of the member viewed from a width direction of the member.
   Substantially the same direction as the discharging direction of the member viewed from the width direction of the member means that there is no above-described predetermined angle in the thickness direction of the member between the discharging direction of the base material remainder and the discharging direction of the member. Therefore, according to the present invention, there is no bending stress in the thickness direction of the base material so that the member can be surely cut out from the base material having small flexural yield strength in the thickness direction.
   Also, when the bending rigidity of the member in the thickness direction is high, the member and the base material remainder can be surely separated by, for example, providing the angle between the discharging direction of the member and the discharging direction of the base material remainder to be about 0 ° viewed from the thickness direction of the member and about 5 ° viewed from the width direction of the member.
(4) The cutter described above, wherein said base material remainder discharging mechanism comprises a suction device for sucking each of the two parts of the base material remainder.
   The base material remainder is divided into two in a direction along the discharging direction of the member. Thus, according to the present invention, the base material remainder can be pulled for discharging thereof even if the tensile strength of the member is low.
(5) The cutter described above, wherein the embossing rate of the side portions of the continuous sheet of base material is 15% or more and the embossing rate of the centre portion thereof is 0.5% to 10%.
   Embossing includes thermal embossing, ultrasonic sealing and the like. Embossing may be arranged, for example, in lines, dots and the like. When the member is used as an absorbent body of an interlabial pad, it is preferable to be in dots in consideration of the comfortableness of the interlabial pad. In this case, embossing on the base material remainder is preferable to be, for example, within the range of 0.3 to 1.0 mm² arranged at 0.5 to 2 mm intervals.
   According to the present invention, the tensile strength increases as the embossing rate increases. Therefore, the tensile strength of the base material remainder can be increased even when the tensile strength of the member is low so that the base material remainder can be surely discharged by pulling.
   Said embossing may be uniformly applied in the continuing direction of the base material.
   According to the present invention, the tensile strength in the continuing direction of the base material can be made uniform so that the base material remainder enables to be prevented from an accidental breaking when the base material remainder is pulled.
   The term "dividing one into two" here includes the state where the base material remainder is visually completely separated into two and the state where it is practically separated into two even though the two parts appear to be in contact.
   The dividing means comprises cutting-plane-line-forming means which enables to divide the base material remainder into two parts by forming a cutting plane line on the base material remainder along the continuing direction thereof.
   The cutting plane line is a slit.
   The cutting plane line may be formed through the member in the thickness direction or formed into a half-way in the thickness direction of the member. The length and the shape of the cutting plane line are not limited, and it may be in a straight line or a curbed line. Also, the number of the cutting plane line is not specifically limited.
(6) The cutter described above, wherein the driving mechanism comprises a roller and a rotating mechanism for rotating said roller, and the cutting blade is a blade provided on the roller; and wherein the rotating mechanism rotates said roller so that the blade presses against the base material, thereby cutting out the member from the base material.
(7) The cutter described above, wherein the member is an absorbent body and said cutting blade has a shape for cutting out the absorbent body.
   It is preferable to form the absorbent body with a material which is bulky and hard to be deformed. Examples of the materials for forming the absorbent body may be pulp, chemical pulp, pulverized pulp, rayon, acetate, natural cotton, air laid pulp to which chemical bonding is applied, super absorbent polymer, super absorbent polymer fiber, synthetic fiber, and a material obtained by mixing them appropriately.
   Specifically, an example of the absorbent body may be a nonwoven sheet with 50 to 250 g/m² and 2 to 5 mm bulkiness, which is obtained by preparing a sheet through entangling fiber, in which 60 to 90 % of rayon or acetate within the range of 1.1 to 6.6 dtex and 10 to 40 % of super absorbent polymer fiber are laminated by this mixing ratio, by needling.
(8) The cutter described above, wherein the cutter is mounted on an apparatus for producing an interlabial pad, having the absorbent body.
(9) A cutting method for cutting out a member by pressing a cutting blade against a continuous sheet of base material, comprising the steps of: conveying the base material along a continuing direction; embossing the continuous sheet of base material such that an embossing rate of side portions of the continuous sheet of base material is higher than an embossing rate of a centre portion in the width direction of the continuous sheet of base material; cutting out the member by pressing the cutting blade against the base material; discharging the member which is cut out by the cutting blade; and dividing a base material remainder, which is a remainder of the continuous sheet of base material after the member is cut out, into two parts along the continuing direction of the continuous sheet of base material, wherein said step of dividing the base material remainder into two parts further comprises the steps of: forming slits as perforation cutting plane lines along substantially a centre of the continuous sheet of base material in the continuing direction by using a blade to press against the continuous sheet of base material and; discharging the two parts of the base material remainder in directions away from each other.
(10) The cutting method described above, wherein, in the discharging step, each of the two parts of the base material remainder is discharged in a thickness direction of the member at a predetermined angle to a discharging direction of the member.
(11) The cutting method described above, wherein, in the discharging step, each of the two parts of the base material remainder is discharged in substantially the same direction as a discharging direction of the member when viewed from the width direction of the member.
(12) The cutting method described above, wherein the embossing rate of the side portions of the continuous sheet of base material is 15% or more and the embossing rate of the centre portion thereof is 0.5% to 10%.
   Said embossing may be uniformly applied on the base material in the continuing direction thereof.
   In the cutting step, the base material remainder is divided into two by forming a slit on the base material remainder along the continuing direction.
(13) The cutting method described above, wherein, in said cutting step, the cutting blade is provided on a roller and said cutting blade is pressed against the base material by rotating the roller so as to cut out the member from the base material.
(14) The cutting method described above, wherein the cutting method is incorporated in a method for producing an interlabial pad.

### BRIEF DESCRIPTION OF THE DRAWINGS

Fig. 1 is a schematic view showing an apparatus for producing an interlabial pad (not an embodiment of the invention).
Fig. 2A is a plan view of an absorbent body in the apparatus for producing an interlabial pad (not an embodiment of the invention).
Fig. 2B is an enlarged plan view of an absorbent body produced in the apparatus for producing the interlabial pad (not an embodiment of the invention).
Fig. 3 is a perspective view of a cutter (not an embodiment of the invention).
Fig. 4A is a side view showing the state where the absorbent body is cut out in the cutter (not an embodiment of the invention) .
Fig. 4B is a side view showing the state where there is a crack generated at the end part of the absorbent body in the cutter (not an embodiment of the invention).
Fig. 4C is a side view showing the state where the crack in the end part of the absorbent body is to be expanded in the cutter (not an embodiment of the invention).
Fig. 4D is a side view showing the state where the crack in the end part of the absorbent body has expanded in the cutter (not an embodiment of the invention).
Fig. 5 is a schematic view of a cut pattern of the cutter (not an embodiment of the invention).
Fig. 6 is a schematic view showing a cut pattern of a cutter (not an embodiment of the invention).
Fig. 7 is a perspective view showing a cutter according to a comparative example.
Fig. 8A is side view showing the state where the absorbent body is cut out in the cutter according to the comparative example.
Fig. 8B is a side view showing the state where there is a crack generated at the end part of the absorbent body in the cutter according to the comparative example.
Fig. 8C is a side view showing the state where the crack in the end part of the absorbent body is to be expanded in the cutter according to the comparative example.
Fig. 8D is a side view showing the state where the absorbent body, together with the base material remainder, moves in the take-up direction in the cutter according to the comparative example.
Fig. 9 is a schematic view showing a cutter (not an embodiment of the present invention).
Fig. 10 is a plan view of a continuous base material according to the cutter of Fig. 9.
Fig. 11 is a perspective view of a cutter (not an embodiment of the invention).
Fig. 12 is a perspective view of a continuous base material produced in a cutter according to an embodiment of the present invention.
Fig. 13 is a perspective view of a cutter (not an embodiment of the invention).

### DETAILED DESCRIPTION

Examples which are useful for the understanding of the invention, but not embodiments of the invention, and embodiments of the present invention will be described hereinafter by referring to the accompanying drawings. In the description of the examples and embodiments below, the same numerals are applied to the same structural elements and the description therefore will be omitted or simplified.

### First Example (not an embodiment of the invention)

### Structure of an apparatus for producing, interlabial pad

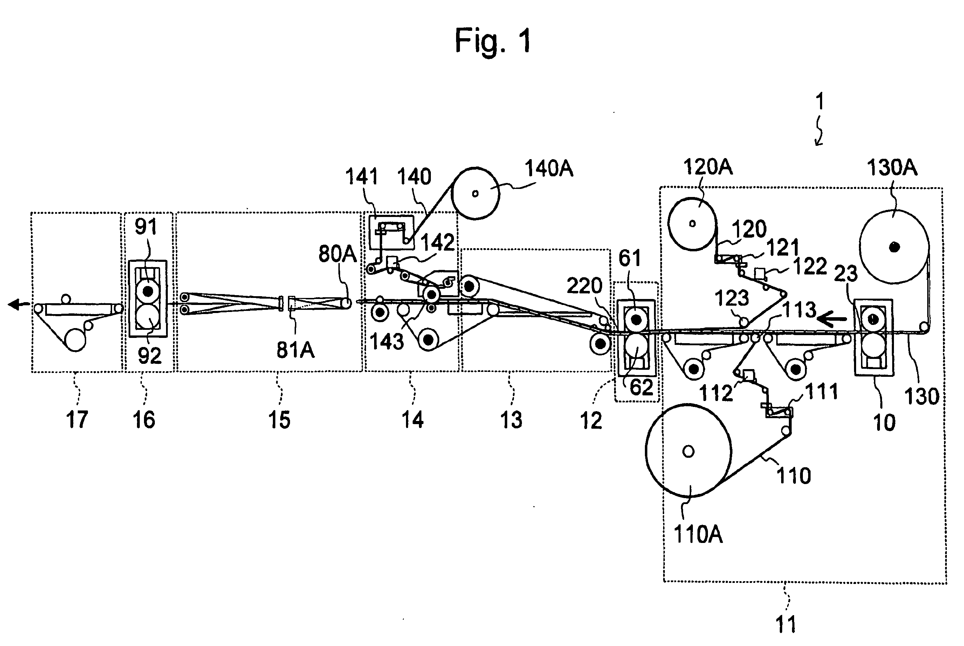

Fig. 1 shows an apparatus for producing an interlabial pad comprising a cutter according to the first example (not an embodiment of the invention).

An interlabial pad producing apparatus 1 includes a raw material supplier 11, a bonding-fixing unit 12, a protruded-area forming unit 13, a mini-sheet piece attaching unit 14, a folding unit 15, a cutting unit 16, and a product conveyer 17.

In the raw material supplier 11, a continuous base material 130 wound on a raw material roller 130A is fed from the rawmaterial roller 130A to a cutter 10. In the cutter 10, absorbent bodies 23 are cut out from the continuous base material 130, and the absorbent bodies 23 are transferred in the arrow direction. The cutter 10 will be described later.

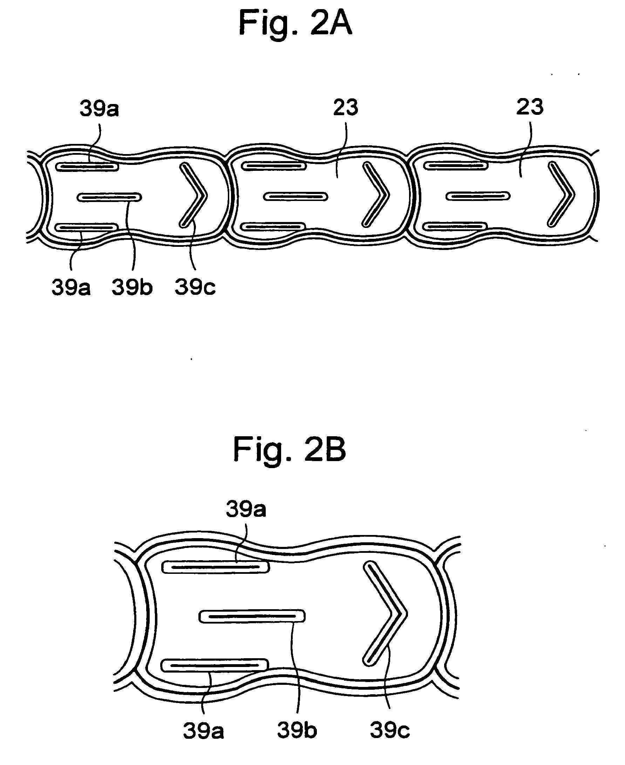

As shown in Fig. 2A and Fig. 2B, four slits 39a, 39b, 39c are formed in the absorbent body 23 by the cutter 10. The center slit 39b is formed so that the absorbent body 23 can be easily bent. The both ends slits 39a are formed so that the mini-sheet piece to be described later can be easily attached. The mountain-shaped slit 39c is formed for fitting fingers of a wearer to the interlabial pad when the pad is worn.

In the raw material supplier 11, a top sheet continuous member 110 wound on a raw material roller 110A is fed to an adhesive-applying device 112 from the raw material roller 110A through a meander-correcting device 111. Then, a hot-melt adhesive is applied onto the top sheet continuous member 110 in the adhesive-applying device 112, and it is sent over a roller 113.

Further, in the raw material supplier 11, a back sheet continuous member 120 wound on a raw material roller 120A is fed to an adhesive-applying device 122 from the raw material roller 120A through a meander-correcting device 121. Then, hot-melt adhesive is applied onto the back sheet continuous member 120 in the adhesive-applying device 122 and it is sent under a roller 123.

Thereby, the top sheet continuous member 110 and the back sheet continuous member 120 sandwich therebetween the absorbent body 23.

In the bonding-fixing unit 12, the top sheet continuous member 110, the back sheet continuous member 120, and the absorbent body 23 are pressed between an upper shaft roller 61 and a lower shaft roller 62. Thereby, the top sheet continuous member 110 and the back sheet continuous member 120 are bonded and fixed with the absorbent body 23 therebetween, and a continuous absorbent layer 220 is formed.

In the protruded-area forming unit 13, the continuous absorbent layer 220 is made into a V-shape toward the downstream. Thus, the absorbent member of the complete interlabial pad has a convex protruded area along the center line.

In the mini-sheet piece attaching unit 14, a mini-sheet piece continuous member 140 wound on a raw material roller 140A is fed to an adhesive-applying device 142 from the raw material roller 140A through a meander-correcting device 141. Then, hot-melt adhesive is applied onto themini-sheet piece continuous member 140 in the adhesive-applying device 142.

The mini-sheet piece continuous member 140 to which the hot-melt adhesive is applied is kept on a roller with suction 143. In this state, it is cut into a predetermined length by a cut roller including a cutting blade.

The mini-sheet piece cut into the predetermined length is sent to the back sheet continuous member 120 side of the continuous absorbent layer 220 and laminated with the continuous absorbent layer 220 being bent in V-letter shape.

A folding unit 15 includes a roller 80A, a roller 81A orthogonally provided on the roller 80A, and a conveyer belt wound around the rollers 80A and 81A.

The continuous absorbent layer 220 is conveyed on the conveyer belt so that both sides of the continuous absorbent layer 220 are folded. Then, the folded continuous absorbent layer 220 gradually is changed to the horizontal position.

The cutting unit 16 includes a lower shaft roller 92, and an upper shaft roller 91 having a cutter blade. In the cutting unit 16, the lower shaft roller 92 and the upper shaft roller 91 press and cut the continuous absorbent layer 220 with mini-sheet piece so as to form an elliptic-shape interlabial pad.

Completed individual interlabial pads formed as described are conveyed by the product conveyer 17 and then go through wrapping and packaging steps for shipment.

### Structure of Cutter

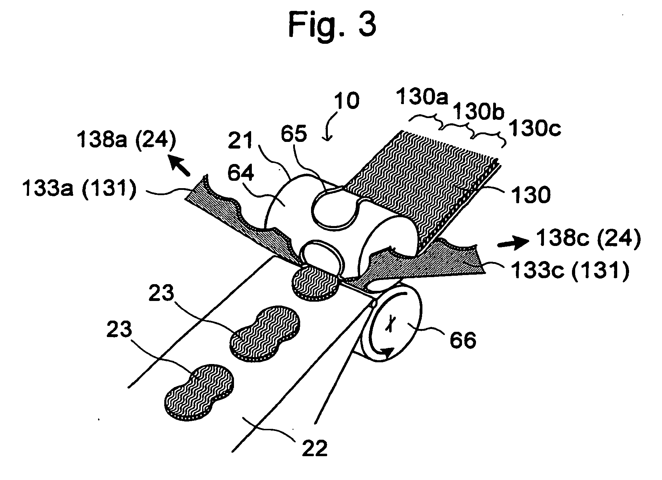

Fig. 3 shows the cutter 10.

The cutter 10 includes a cutting mechanism 21 for cutting out the absorbent bodies 23 from the continuous base material 130 as well as for forming trim absorbent bodies 133a and 133c by dividing a base material remainder 131, which is the remainder of the continuous base material 130 after the absorbent body 23 is cut out thereof, into two. Also, the cutter 10 includes a transporting conveyer 22 as member discharging means for discharging the absorbent bodies 23 and a base material remainder discharging mechanism 24 as base material remainder discharging means for discharging each of the trim absorbent bodies 133a and 133c toward the directions away from each other.

The cutting mechanism 21 includes a cutting roller 64, a driving motor (not shown) as a driving mechanism for rotating the cutting roller 64, and a backing roller 66 arranged at a predetermined space between the cutting roller 64. Here, the cutting roller 64, and driving motor, comprises the driving mechanism described in the claim. Also, the cutting mechanism 21, comprises the means for conveying, means for cutting, and means for dividing.

A cutting blade 65 having the edge at the end portion protrudes from the cutting roller 64. Also, inside the cutting blade 65, a sponge with high rebound elasticity is provided partially or entirely.

The backing roller 66 is pressed against the cutting roller 64 with a predetermined pressure, and the surface of the backing roller 66 is made soft so as not to damage the edge of the cutting blade 65. The rotation of the backing roller 66 synchronizes with the rotation of the cutting roller 64 so that no shear force occurs in the cutting blade 65.

The continuous base material 130 is a continuous sheet and includes center part 130b, and two side parts 130a, 130c in the width direction.

The continuous base material 130 wound on the raw material roller 130A (see Fig. 1) is supported by the raw material roller 130A at its one end and pinched between the cutting roller 64 and the backing roller 66 at the other end, and placed a predetermined tension on thereto.

The base material remainder discharging mechanism 24 includes a suction device (not shown) for sucking each of the trim absorbent bodies 133a, 133c in the directions of arrows 138a and 138c shown in Fig. 3, and take-up devices (not shown) for taking up each of the trim absorbent bodies, respectively, which are sucked by the suction device.

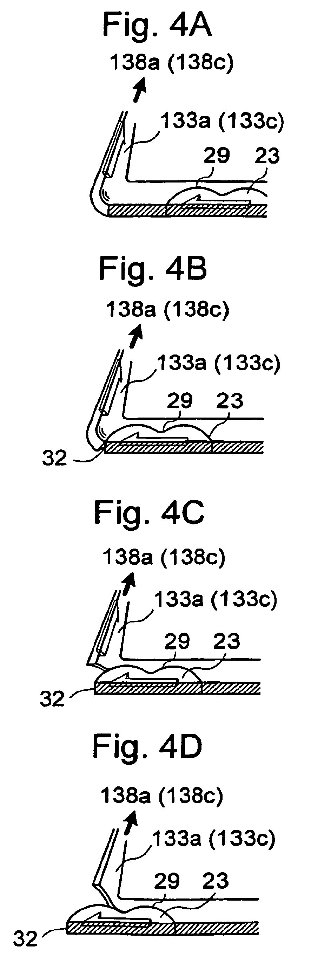

With the cutter 10 as described, upon starting the driving motor, the cutting roller 64 rotates and the continuous base material 130 is conveyed from the raw material roller 130A toward the cutting device 10 along its continuing direction. At the same time, due to the rotation of the cutting roller 64, the cutting blade 65 approaches substantially the center of the continuous base material 130 in the width direction to press against the continuous base material 130. Thereby, there is a cutting plane line 29 (see Fig. 4A to Fig. 4D) formed on the continuous base material 130 by the cutting blade 65 of the cutting roller 64, and the absorbent body 23 as a member in a predetermined shape is cut out from the center part 130b. The absorbent body 23 is pushed out by the rotation of the cutting roller 64 and conveyed on the transporting conveyer 22.

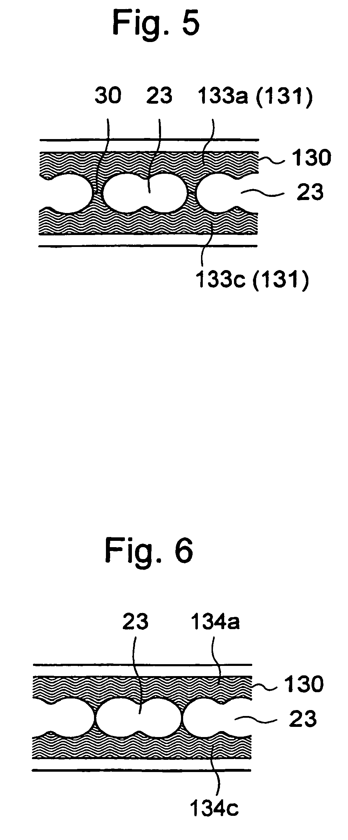

On the other hand, the base material remainder 131, which is the remainder of the continuous base material 130 after the absorbent body 23 is cut out, is cut into two in the continuing direction of the continuous base material 130 by the cutting plane lines 29, 30 (see Fig. 5) formed by the cutting blade 65, and thereby formed into the trim absorbent bodies 133a, 133c. The trim absorbent bodies 133a, 133c are discharged by the base material remainder discharging mechanism 24 in the directions away from each other, that is, in the directions of arrows 138a, 138c.

The directions of taking up the trim absorbent bodies 133a, 133c (directions of arrows 138a, 138c) by the base material remainder discharging mechanism 24 and the discharging direction of absorbent body 23 make a predetermined angle in the thickness direction of the absorbent body 23. Specifically, the predetermined angle may be within the range of 0° to 45°. With this, the absorbent body 23 is not to interfere with the trim absorbent bodies 133a, 133c much so that it is possible to suppress the dislocation.

Operation of the cutter 10 when cutting out the absorbent body 23 from the continuous base material 130 will be described in detail by referring to Fig. 4A to Fig. 4D.

First, the absorbent body 23 is cut out in the shape of the absorbent body along the cutting plane line 29 by the cutting blade 65. In this state, the absorbent body 23 is not separated from the trim absorbent bodies 133a, 133c (Fig. 4A).

Next, the end portion of the absorbent body 23 begins to be discharged from the cutting mechanism 21. At the same time, each of the trim absorbent bodies 133a, 133c, which are the remainder of the continuous base material after the absorbent body 23 is cut out, are brought out in the directions of the arrows 138a, 138c respectively. The force for pulling out each of the trim absorbent bodies 133a, 133c works on the end portion of the absorbent body 23, so that a crack 32 occurs in the bottom tip of the end portion of the absorbent body 23 (Fig. 4B).

The crack 32 expands along the cutting plane line 29 and the absorbent body 23 begins to be separated (Fig. 4C and Fig. 4D) . As a result, the absorbent body 23 is completely separated from the continuous base material 130.

The example, as shown in Fig. 5, adopts a cut pattern formed from the absorbent bodies 23 on the continuous base material 130 at predetermined intervals. Here, a cutting plane line 30 is formed between the adjacent ends of the absorbent bodies 23.

Thereby, the base material remainder 131 can be divided easily into the trim absorbent bodies 133a, 133c. Also, the cutting plane line forming the end portion and the cutting planes line forming the other end portion of the absorbent bodies 23 do not cross each other. Thus, the amount of scraps generated, when the absorbent bodies 23 is cut out, can be decreased.

With the example, the following effects can be expected.

The cutter 10 is formed including the cutting mechanism 21, the transporting conveyer 22, and the base material remainder discharging mechanism 24. Thereby, the cutter 10 divides the base material remainder 131, which is the remainder of the continuous base material 130 after the absorbent body 23 is cut out, into two for forming the two trim absorbent bodies 133a, 133c, and separates the trim absorbent bodies 133a, 133c from each other. Therefore, the trim absorbent bodies 133a, 133c do not interfere with the end of the absorbent body 23. As a result, the absorbent bodies 23 are smoothly brought out by the transporting conveyer 22 from the two rollers 64 and 66. Thus, the dislocations of the absorbent bodies 23 can be prevented

Since a sponge is provided in the inner side of the cutting blade 65 of the cutting roller 64, rebound elasticity of the sponge works when the cutting roller 64 is pressed against the continuous base material 130. Thus, the pressing force of the cutting blade 65 against the continuous base material 130 can be made uniform and the absorbent bodies 23 can be smoothly cut out.

Also, with the interlabial pad producing apparatus 1, the absorbent bodies 23 for forming the interlabial pads can be stably produced from the continuous base material 130.

### Second Example (not an embodiment of the invention)

Fig. 6 shows the cut pattern of a cutter according to the second example (not an embodiment of the invention). The example differs from the first example in respect of the cut pattern formed from the absorbent bodies 23 continuously on the continuous base material 130.

With the example, the amount of the trim taken up in trim absorbent bodies 134a, 134c can be decreased.

### Comparison Example

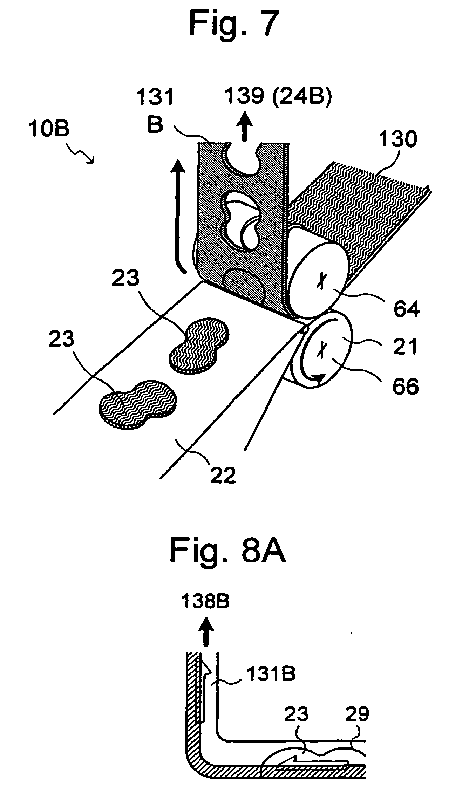

Fig. 7 shows a cutter 10B according to a comparison example.

In the comparison example, the structure of a base material remainder discharging mechanism 24B is different from that of the first example.

Specifically, in the comparison example, the base material remainder discharging mechanism 24B does not divide a base material remainder 131B into two but sucks it to the direction of an arrow 139 shown in Fig. 7, that is, in the orthogonal direction to the discharging direction of the absorbent bodies 23.

Operation of the cutter 10B for cutting out the absorbent body 23 of the continuous base material 130 will be described in detail by referring to Fig. 8A to Fig. 8D.

First, the continuous base material 130 is cut out along the cutting plane line 29 by the cutting blade 65 in the shape of the absorbent body 23 (Fig. 8A).

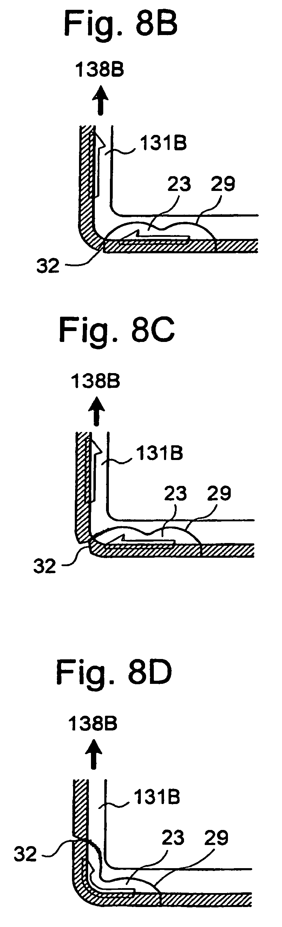

Next, the end portion of the absorbent body 23 begins to be discharged from the cutting mechanism 21. At the same time, the base material remainder 131B, which is the remainder of the continuous base material 130 after the absorbent body 23 is cut out, is brought out in the direction of the arrow 139. The force for pulling out each of the base material remainder 131B works on the top tip end portion of the absorbent body 23, so that a crack 32 occurs in the bottom tip of the end portion of the absorbent body 23 (Fig. 8B).

The crack 32 is to expand along the cutting plane line 29. However, the end portion of the absorbent body 23 interferes with the end edge of the base material remainder 131B. Thus, the end portion of the absorbent body 23 clings to the end edge of the base material remainder 131B so as to be brought toward the direction of the arrow 139 together with the base material remainder 131B (Fig. 8C and Fig. 8D). As a result, the absorbent body 23 cannot be completely separated from the continuous base material 130.

### Third example

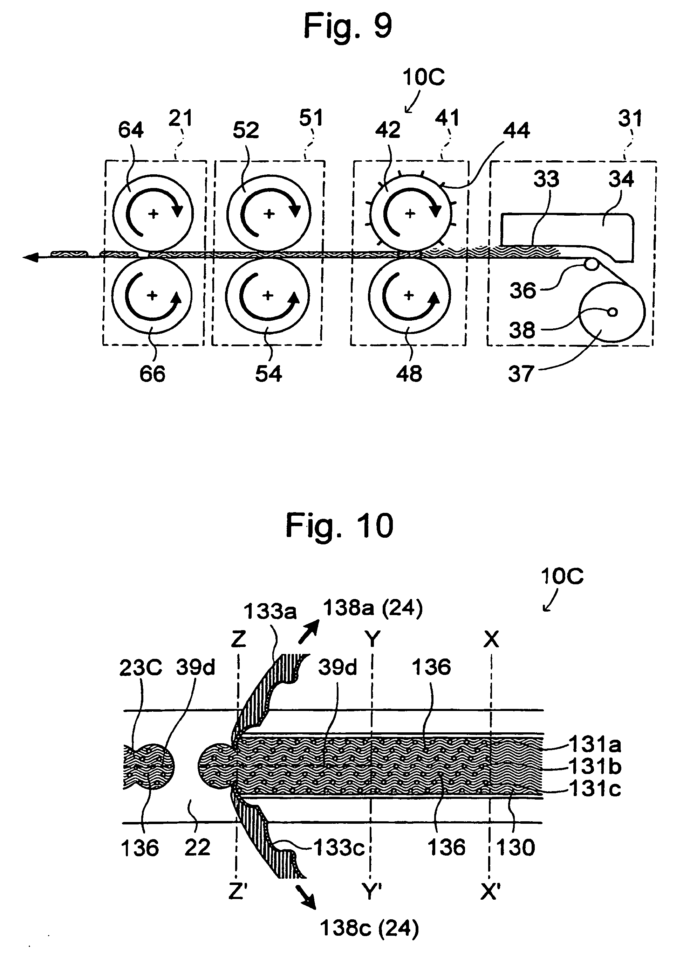

Fig. 9 and Fig. 10 show a cutter 10C not according to the present invention.

The example differs from the first example in respect that it includes an embossing device 41 and a slit forming device 51.

The cutter 10C includes the cutting mechanism 21, the transporting conveyer 22, and the base material remainder discharging mechanism 24. The cutter 10C further includes the embossing device 41 and the slit forming device 51 as a cutting plane line forming means. The embossing device 41 is provided along the line X-X' shown in Fig. 10, the slit forming device 51 is provided along the line Y-Y' shown in Fig. 10, and the cutting mechanism 21 is provided along the line Z-Z'.

In the example, the continuous base material 130 is fed by the sheet fabric supplier 31. The sheet fabric supplier 31 is for forming the continuous base material 130 and includes a spool 38 on which a sheet fabric 37 for the absorbent body is wound, a roller 36, and an opening/laminating device 34 for supplying the absorbent material.

The embossing device 41 is for embossing the continuous base material 130 and includes an embossing roller 42 having protruded parts for forming dented embossed parts 136 on the continuous base material 130, as well as an embossing backing roller 48 having a smooth surface.

The slit forming device 51 includes a slit roller 52 having a blade and a backing roller 54 having a smooth surface. The slit forming device 51 forms slits as perforation cutting plane lines in the substantially center along the continuing direction of the continuous base material 130 by pressing the slit roller 52 thereto. Moreover, the present invention provides, the cutting device mechanism 21, comprises by means for conveying and means for cutting, and the slit forming device 51 and the base material remainder discharging mechanism 24 comprises dividing means in the claims.

The sheet fabric 37 used for the absorbent body is fed to the opening/laminating device 34 through the spool 38 and the roller 36. The absorbent material is supplied from a supply port 33 of the opening/laminating device 34 on top of the sheet fabric 37. The absorbent material is opened and the coating material is laminated, so that the continuous base material 130 is formed.

Then, the continuous base material 130 is embossed by the embossing device 41.

Subsequently, the continuous base material 130 is supplied in between the slit roller 52 and the backing roller 54, and a perforation slit 39d is formed thereon in substantially the center of width direction in the continuing direction of the continuous base material 130.

Then, absorbent bodies 23C are cut out from the continuous base material 130 by the cutter 10 and the absorbent bodies 23C are discharged as they are. At the same time, the base material remainder 131 is pulled by the base material remainder discharging mechanism 24 in the directions of the arrow 138a, 138c. Thus, the base material remainder 131 is torn along the slit 39d to be divided into the trim absorbent bodies 133a, 133c and pulled out.

The perforation slit 39d is formed in substantially the center of the continuous base material 130 in the continuing direction. Thus, the interlabial pad can be folded along the slit 39d as to improve the fitness to the inner wall of the labium.

Further, embossing can improve the tensile strength of each of the trim absorbent bodies 133a, 133c. Therefore, each of the trim absorbent bodies 133a, 133c can be surely pulled out by the suction device.

In consideration of suppressing the deformation of the interlabial pad when worn and maintaining the softness, the embossing rate of the absorbent body 23C per area is preferable to be within the range of 0.5 to 10 % and more preferable to be 1.0 to 5.0 %. In consideration of suppressing the breakage of the trim absorbent bodies 133a, 133c, the embossing rate of the trim absorbent bodies 133a, 133c per area is preferable to be 15 % or more and more preferable to be 40 % or more.

### Fourth Example (not an embodiment of the invention)

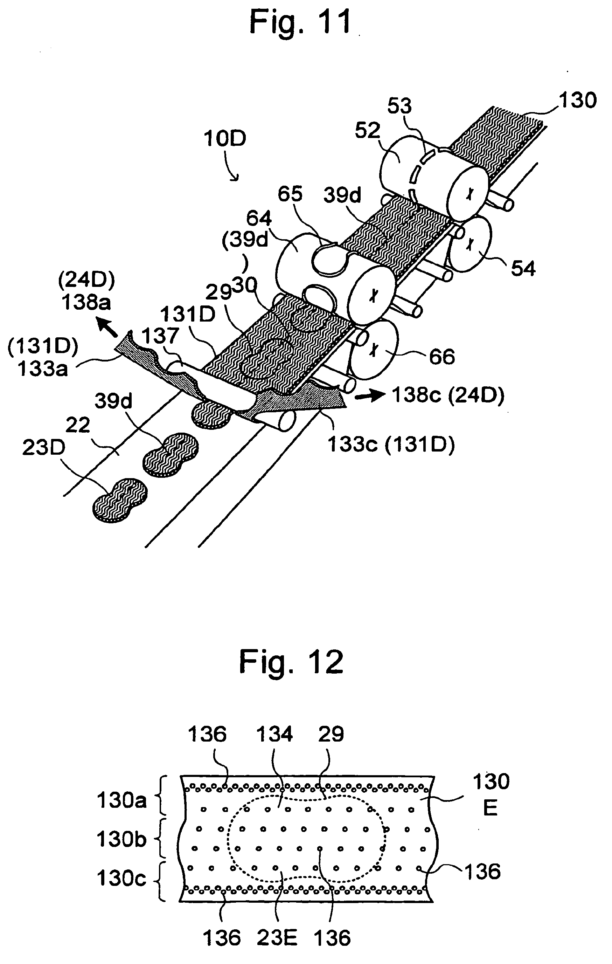

Fig. 11 shows a cutter 10D according to the fourth example (not an embodiment of the invention).

The example differs from the third example in the respect that it does not include an embossing device. Also, the example differs in respect of the structure of a base material remainder discharging mechanism 24D.

Specifically, after absorbent bodies 23D are cut out by the cutting mechanism 21, the absorbent bodies 23D and the base material remainder 131D are discharged by the transporting conveyer 22. Then, on the roller 137, the base material remainder 131D is pulled in the directions of the arrows 138a, 138c to be divided into two trim absorbent bodies 133a, 133c and pulled out.

In the example, the absorbent bodies 23D are discharged along the continuing direction of the continuous base material 130 after the absorbent bodies 23D are cut out, so that dislocations can be further suppressed.

### Embodiment of the invention

Fig. 12 shows a continuous base material 130E which is processed by a cutter according to an embodiment of the present invention.

The embodiment differs from the fourth example in respect to the embossing rate of the continuous base material 130E.

Specifically, the embossing rate of side portions 130a, 130c of the continuous base material 130E is higher than that of the center portion 130b, and the embossed portion 136 is uniformly formed along the continuing direction of the continuous base material 130E. Therefore, the embossing rate of the trim absorbent body becomes higher than that of the absorbent body 23E.

Thereby, the strength of the trim absorbent body can be improved, so that it enables to prevent the breakage of the trim absorbent body when the trim absorbent body is being pulled.

Also, even though plural numbers of sheets are laminated to form the continuous base material 130E, the plural numbers of sheets are unified by embossment. Thus, the trim absorbent body can be surely pulled out.

Examples of the absorbent body 23E may be rayon, acetate, natural cotton, super absorbent polymer fiber, synthetic fiber, pulp, chemical pulp, and mixtures of them. In consideration of softness of the absorbent body, the absorbent body is preferable to be an aggregate of fibers which mainly contains rayon with the fiber length of 10 to 51 mm. In this case, the pitch of the embossment in the embossed portion 136 is preferable to be within the range of 1 to 9 mm.

### Fifth example (not an embodiment of the invention)

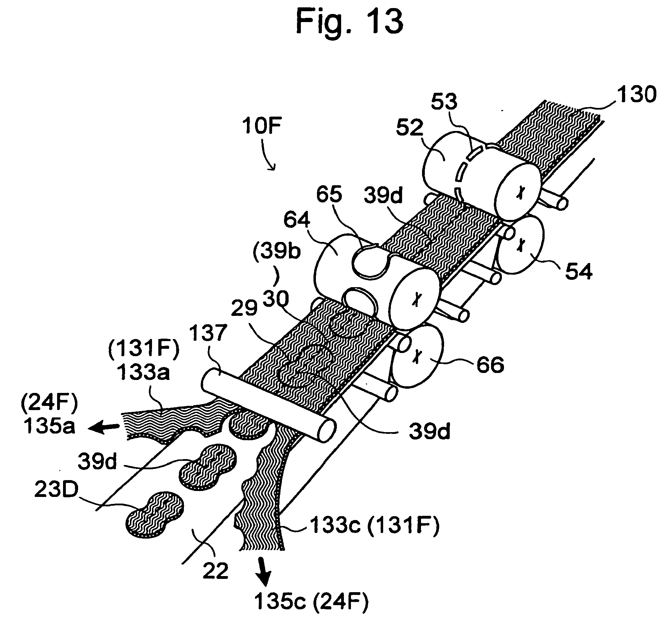

Fig. 13 shows a cutter 10F according to a fifth example (not an embodiment of the invention).

The example differs from the fourth example in respect of the structure of a base material remainder discharging mechanism 24F.

Specifically, a base material remainder 131F is pulled in the directions of 135a, 135c on the roller 137 so as to be divided into two trim absorbent bodies 133a, 133c and pulled out. The directions of the arrows 135a, 135c are substantially the same directions as the discharging direction of the absorbent bodies 23 viewed from the width directions of the absorbent body 23D, that is, in the horizontally expanding directions.

In the example, the absorbent bodies 23D are not pulled in the thickness direction, so that dislocations of the absorbent bodies 23D can be more surely suppressed.

The present invention is not limited to the above-described embodiments but it includes various modifications and improvements which achieve the object of the invention.

In the production lines of the interlabial pad producing apparatus 1, the speed of the line may be set to be about 20 to 200 m/min. and the production rate of the absorbent bodies may be set to be about 200 to 2000 pieces per minute.

In the first example, the tensile strength of the side portions 130a, 130c of the continuous base material 130 in the continuing direction, from which the trim absorbent bodies 133a and 133c are formed, may be set larger than that of the center portion 130b from which the absorbent bodies 23 are formed. with this, rigidity of the trim absorbent body can be increased while the softness of the absorbent bodies 23 is maintained. Also, the trim absorbent bodies 133a, 133c enable to prevent breakage when pulled out.

According to the cutter and cutting method, the member and the base material remainder can be surely separated. Thus, the dislocations of the cut-out members can be prevented.

## Claims

1. A cutter (10) for cutting out a member (23) from a continuous sheet of base material(130), comprising:
a conveying means (21, 41, 51, 52) for conveying the continuous sheet of base material (130) along a continuing direction of the continuous sheet of base material(130);
a cutting means (21), having a cutting blade (65) and a driving mechanism for bringing said cutting blade (65) into substantially a centre portion (130b) in a width direction of the base material (130) being conveyed, for cutting out the member (23) by pressing said cutting blade (65) against the base material(130);
a member discharging means (22) for discharging the member (23) which is cut out by the cutting means (21); and
a dividing means (24) for dividing a remainder (131) of the continuous sheet of base material(130) after the member (23) is cut out into two parts (133a, 133c) along the continuing direction of the continuous sheet of base material (130) such that the remainder (131) of the base material (130) is separated from the member (23);
**characterised in that** said dividing means (24) comprises a slit forming device (51) having a blade (53) which forms slits (39d) as perforation cutting plane lines along substantially a centre of the continuous sheet of base material(130) in the continuing direction;
and **in that** said cutter further comprises: a base material remainder discharging mechanism (24) for discharging each of the two parts (133a, 133c) of the base material remainder (131) in directions away from each other; and
an embossing means (41) for embossing the continuous sheet of base material (130) wherein said embossing means (41) embosses the continuous sheet of base material (130) such that an embossing rate of side portions (130a, 130c) of the continuous sheet of base material (130) is higher than an embossing rate of the centre portion (130b) of the continuous sheet of base material (130) in the width direction of the continuous sheet of base material (130).

2. The cutter (10) according to claim 1, wherein said base material remainder discharging mechanism (24) discharges each of the two parts (133a, 133c) of the base material remainder (131) in a thickness direction of the member (23) at a predetermined angle to a discharging direction of the member (23).

3. The cutter (10) according to claim 1, wherein said base material remainder discharging mechanism (24) discharges each of the two parts (133a, 133c) of the base material remainder (131) in substantially the same direction as a discharging direction of the member (23) when viewed from a width direction of the member (23).

4. The cutter (10) according to any one of claims 1 to 3, wherein said base material remainder discharging mechanism (24) comprises a suction device for sucking each of the two parts of the base material remainder (131).

5. The cutter (10) according to any one of claims 1 to 4, wherein the embossing rate of the side portions (130a, 130c) of the continuous sheet of base material (130) is 15% or more and the embossing rate of the centre portion (130b) thereof is 0.5% to 10%.

6. The cutter (10) according to any one of claims 1 to 5, wherein said driving mechanism comprises a roller (64) and a rotating mechanism for rotating said roller (64), and said cutting blade (65) is a blade provided on said roller(64); and
wherein said rotating mechanism rotates said roller(64) so that said blade (65) presses against the base material (130), thereby cutting out the member (23) from the base material(130).

7. The cutter (10) according to any one of claims 1 to 6, wherein said member (23) is an absorbent body and said cutting blade (65) has a shape for cutting out the absorbent body.

8. The cutter (10) according to claim 7, wherein the cutter (10) is mounted on an apparatus (1) for producing an interlabial pad having the absorbent body.

9. A cutting method for cutting out a member (23) by pressing a cutting blade (65) against a continuous sheet of base material (130), comprising the steps of:
conveying the base material (130) along a continuing direction;
embossing the continuous sheet of base material (130) such that an embossing rate of side portions (130a, 130c) of the continuous sheet of base material (130) is higher than an embossing rate of a centre portion (130b) in the width direction of the continuous sheet of base material (130);
cutting out the member (23) by pressing said cutting blade (65) against the base material (130);
discharging the member (23) which is cut out by the cutting blade (65); and
dividing a base material remainder (131), which is a remainder of the continuous sheet of base material (130) after the member (23) is cut out, into two parts (133a, 133c) along the continuing direction of the continuous sheet of base material (130), wherein said step of dividing the base material remainder (131) into two parts (133a, 133c) further comprises the steps of:
forming slits as perforation cutting plane lines along substantially a centre of the continuous sheet of base material (130) in the continuing direction by using a blade (53) to press against the continuous sheet of base material(130); and
discharging the two parts (133a, 133c) of the base material remainder (131) in directions away from each other.

10. The cutting method according to claim 9 wherein, in said discharging step, each of the two parts (133a, 133c) of the base material remainder (131) is discharged in a thickness direction of the member (23) at a predetermined angle to a discharging direction of the member (23).

11. The cutting method according to claim 9 wherein, in the discharging step, each of the two parts (133a, 133c) of the base material remainder (131) is discharged in substantially the same direction as a discharging direction of the member (23) when viewed from the width direction of the member (23).

12. The cutting method according to any one of claims 9 to 11, wherein the embossing rate of the side portions (130a, 130c) of the continuous sheet of base material (130) is 15% or more and the embossing rate of the centre portion (130b) thereof is 0.5% to 10%.

13. The cutting method according to any one of claims 9 to 12 wherein, in said cutting step, said cutting blade (65) is provided on a roller (64) and said cutting blade(65) is pressed against the base material (130) by rotating said roller (64) so as to cut out the member (23) from the base material (130).

14. The cutting method according to any one of claims 9 to 13, wherein the cutting method is incorporated in a method for producing an interlabial pad.

## Patentansprüche

1. Eine Schneideeinrichtung (10) zum Herausschneiden eines Elements (23) aus einer kontinuierlichen Schicht aus Basismaterial (130), die Folgendes beinhaltet:
ein Beförderungsmittel (21, 41, 51, 52) zum Befördern der kontinuierlichen Schicht aus Basismaterial (130) entlang einer fortlaufenden Richtung der kontinuierlichen Schicht aus Basismaterial (130);
ein Schneidemittel (21), das eine Schneideklinge (65) und einen Antriebsmechanismus zum Bringen der Schneideklinge (65) in im Wesentlichen einen mittleren Abschnitt (130b) in einer Breitenrichtung des beförderten Basismaterials (130) aufweist, um das Element (23) durch das Drücken der Schneideklinge (65) gegen das Basismaterial (130) herauszuschneiden;
ein Elementabgabemittel (22) zum Abgeben des Elements (23), das durch das Schneidemittel (21) herausgeschnitten wird; und
ein Trennmittel (24) zum Trennen eines Rests (131) der kontinuierlichen Schicht aus Basismaterial (130) entlang der fortlaufenden Richtung der kontinuierlichen Schicht aus Basismaterial (130) in zwei Teile (133a, 133c), nachdem das Element (23) herausgeschnitten worden ist, so dass der Rest (131) des Basismaterials (130) von dem Element (23) abgeteilt wird,
**dadurch gekennzeichnet, dass** das Trennmittel (24) eine Schlitz bildende Vorrichtung (51) mit einer Klinge (53) beinhaltet, die Schlitze (39d) als ebene Perforationsschnittlinien entlang im Wesentlichen einer Mitte der kontinuierlichen Schicht aus Basismaterial (130) in der fortlaufenden Richtung bildet;
und dass die Schneideeinrichtung ferner Folgendes beinhaltet:
einen Basismaterialrestabgabemechanismus (24) zum Abgeben jedes der zwei Teile (133a, 133c) des Basismaterialrests (131) in auseinander gehenden Richtungen; und
ein Prägemittel (41) zum Prägen der kontinuierlichen Schicht aus Basismaterial (130), wobei das Prägemittel (41) die kontinuierliche Schicht aus Basismaterial (130) so prägt, dass eine Prägerate von Seitenabschnitten (130a, 130c) der kontinuierlichen Schicht aus Basismaterial (130) größer als eine Prägerate des mittleren Abschnitts (130b) der kontinuierlichen Schicht aus Basismaterial (130) in der Breitenrichtung der kontinuierlichen Schicht aus Basismaterial (130) ist.

2. Schneideeinrichtung (10) gemäß Anspruch 1, wobei der Basismaterialrestabgabemechanismus (24) jeden der zwei Teile (133a, 133c) des Basismaterialrests (131) in einer Dickerichtung des Elements (23) in einem vorbestimmten Winkel zu einer Abgaberichtung des Elements (23) abgibt.

3. Schneideeinrichtung (10) gemäß Anspruch 1, wobei der Basismaterialrestabgabemechanismus (24) jeden der zwei Teile (133a, 133c) des Basismaterialrests (131) in im Wesentlichen der gleichen Richtung wie einer Abgaberichtung des Elements (23), wenn von einer Breitenrichtung des Elements (23) betrachtet, abgibt.

4. Schneideeinrichtung (10) gemäß einem der Ansprüche 1 bis 3, wobei der Basismaterialrestabgabemechanismus (24) eine Ansaugvorrichtung zum Ansaugen von jedem der zwei Teile des Basismaterialrests (131) beinhaltet.

5. Schneideeinrichtung (10) gemäß einem der Ansprüche 1 bis 4, wobei die Prägerate der Seitenabschnitte (130a, 130c) der kontinuierlichen Schicht aus Basismaterial (130) 15 % oder mehr beträgt und die Prägerate des mittleren Abschnitts (130b) davon 0,5 % bis 10 % beträgt.

6. Schneideeinrichtung (10) gemäß einem der Ansprüche 1 bis 5, wobei der Antriebsmechanismus eine Rolle (64) und einen Drehmechanismus zum Drehen der Rolle (64) beinhaltet und die Schneideklinge (65) eine auf der Rolle (64) bereitgestellte Klinge ist; und
wobei der Drehmechanismus die Rolle (64) so dreht, dass die Klinge (65) gegen das Basismaterial (130) drückt, wodurch das Element (23) aus dem Basismaterial (130) herausgeschnitten wird.

7. Schneideeinrichtung (10) gemäß einem der Ansprüche 1 bis 6, wobei das Element (23) ein absorbierender Körper ist und die Schneideklinge (65) eine Form zum Herausschneiden des absorbierenden Körpers aufweist.

8. Schneideeinrichtung (10) gemäß Anspruch 7, wobei die Schneideeinrichtung (10) auf einem Gerät (1) zum Produzieren eines interlabialen Kissens, das den absorbierenden Körper aufweist, montiert ist.

9. Ein Schneideverfahren zum Herausschneiden eines Elements (23) durch das Drücken einer Schneideklinge (65) gegen eine kontinuierliche Schicht aus Basismaterial (130), das die folgenden Schritte beinhaltet:
Befördern des Basismaterials (130) entlang einer fortlaufenden Richtung;
Prägen der kontinuierlichen Schicht aus Basismaterial (130), so dass eine Prägerate der Seitenabschnitte (130a, 130c) der kontinuierlichen Schicht aus Basismaterial (130) größer als eine Prägerate eines mittleren Abschnitts (130b) in der Breitenrichtung der kontinuierlichen Schicht aus Basismaterial (130) ist;
Herausschneiden des Elements (23) durch das Drücken der Schneideklinge (65) gegen das Basismaterial (130);
Abgeben des Elements (23), das durch die Schneideklinge (65) herausgeschnitten wird; und
Trennen eines Basismaterialrests (131), der ein Rest der kontinuierlichen Schicht aus Basismaterial (130) ist, nachdem das Element (23) herausgeschnitten worden ist, in zwei Teile (133a, 133c) entlang der fortlaufenden Richtung der kontinuierlichen Schicht aus Basismaterial (130), wobei der Schritt des Trennens des Basismaterialrests (131) in zwei Teile (133a, 133c) ferner die folgenden Schritte beinhaltet:
Bilden von Schlitzen als ebene Perforationsschnittlinien entlang im Wesentlichen einer Mitte der kontinuierlichen Schicht aus Basismaterial (130) in der fortlaufenden Richtung durch Verwendung einer Klinge (53) zum Drücken gegen die kontinuierliche Schicht aus Basismaterial (130); und
Abgeben der zwei Teile (133a, 133c) des Basismaterialrests (131) in auseinander gehende Richtungen.

10. Schneideverfahren gemäß Anspruch 9, wobei in dem Abgabeschritt jeder der zwei Teile (133a, 133c) des Basismaterialrests (131) in einer Dickerichtung des Elements (23) in einem vorbestimmten Winkel zu einer Abgaberichtung des Elements (23) abgegeben wird.

11. Schneideverfahren gemäß Anspruch 9, wobei in dem Abgabeschritt jeder der zwei Teile (133a, 133c) des Basismaterialrests (131) in im Wesentlichen der gleichen Richtung wie einer Abgaberichtung des Elements (23), wenn von der Breitenrichtung des Elements (23) betrachtet, abgegeben wird.

12. Schneideverfahren gemäß einem der Ansprüche 9 bis 11, wobei die Prägerate der Seitenabschnitte (130a, 130c) der kontinuierlichen Schicht aus Basismaterial (130) 15 % oder mehr beträgt und die Prägerate des mittleren Abschnitts (130b) davon 0,5% bis 10 % beträgt.

13. Schneideverfahren gemäß einem der Ansprüche 9 bis 12, wobei bei dem Schneideschritt die Schneideklinge (65) auf einer Rolle (64) bereitgestellt wird und die Schneideklinge (65) durch das Drehen der Rolle (64) gegen das Basismaterial (130) gedrückt wird, um das Element (23) aus dem Basismaterial (130) herauszuschneiden.

14. Schneideverfahren gemäß einem der Ansprüche 9 bis 13, wobei das Schneideverfahren in einem Verfahren zum Produzieren eines interlabialen Kissens eingeschlossen ist.

## Revendications

1. Un organe de coupe (10) pour découper un élément (23) dans une feuille continue de matériau de base (130), comprenant :
un moyen de transport (21, 41, 51, 52) pour transporter la feuille continue de matériau de base (130) suivant une direction constante de la feuille continue de matériau de base (130) ;
un moyen de coupe (21), présentant une lame de coupe (65) et un mécanisme d'entraînement pour amener ladite lame de coupe (65) dans substantiellement une portion centrale (130b) dans une direction en largeur du matériau de base (130) qui est transporté, pour découper l'élément (23) en appuyant ladite lame de coupe (65) contre le matériau de base (130) ;
un moyen de déchargement d'élément (22) pour décharger l'élément (23) qui est découpé par le moyen de coupe (21) ; et
un moyen de division (24) pour diviser un restant (131) de la feuille continue de matériau de base (130) après que l'élément (23) est découpé en deux parties (133a, 133c) suivant la direction constante de la feuille continue de matériau de base (130) de façon à ce que le restant (131) du matériau de base (130) soit séparé de l'élément (23) ;
**caractérisé en ce que** ledit moyen de division (24) comprend un dispositif de formation de fentes (51) présentant une lame (53) qui forme des fentes (39d) sous forme de lignes planes de coupe par perforation le long substantiellement d'un centre de la feuille continue de matériau de base (130) dans la direction constante ;
et **en ce que** ledit organe de coupe comprend en outre :
un mécanisme de déchargement de restant de matériau de base (24) pour décharger chacune des deux parties (133a, 133c) du restant de matériau de base (131) dans des directions qui s'éloignent l'une de l'autre ; et
un moyen de gaufrage (41) pour gaufrer la feuille continue de matériau de base (130), ledit moyen de gaufrage (41) gaufrant la feuille continue de matériau de base (130) de façon à ce qu'un taux de gaufrage de portions latérales (130a, 130c) de la feuille continue de matériau de base (130) soit plus élevé qu'un taux de gaufrage de la portion centrale (130b) de la feuille continue de matériau de base (130) dans la direction en largeur de la feuille continue de matériau de base (130).

2. L'organe de coupe (10) selon la revendication 1, dans lequel ledit mécanisme de déchargement de restant de matériau de base (24) décharge chacune des deux parties (133a, 133c) du restant de matériau de base (131) dans une direction en épaisseur de l'élément (23) à un angle prédéterminé par rapport à une direction de déchargement de l'élément (23).

3. L'organe de coupe (10) selon la revendication 1, dans lequel ledit mécanisme de déchargement de restant de matériau de base (24) décharge chacune des deux parties (133a, 133c) du restant de matériau de base (131) dans substantiellement la même direction qu'une direction de déchargement de l'élément (23) lorsque vu depuis une direction en largeur de l'élément (23).

4. L'organe de coupe (10) selon une quelconque des revendications 1 à 3, dans lequel ledit mécanisme de déchargement de restant de matériau de base (24) comprend un dispositif d'aspiration pour aspirer chacune des deux parties du restant de matériau de base (131).

5. L'organe de coupe (10) selon une quelconque des revendications 1 à 4, dans lequel le taux de gaufrage des portions latérales (130a, 130c) de la feuille continue de matériau de base (130) est de 15 % ou plus et le taux de gaufrage de la portion centrale (130b) de celle-ci est de 0,5 % à 10 %.

6. L'organe de coupe (10) selon une quelconque des revendications 1 à 5, dans lequel ledit mécanisme d'entraînement comprend un rouleau (64) et un mécanisme de rotation pour faire tourner ledit rouleau (64), et ladite lame de coupe (65) est une lame prévue sur ledit rouleau (64) ; et
dans lequel ledit mécanisme de rotation fait tourner ledit rouleau (64) de façon à ce que ladite lame (65) appuie contre le matériau de base (130), découpant de ce fait l'élément (23) dans le matériau de base (130).

7. L'organe de coupe (10) selon une quelconque des revendications 1 à 6, dans lequel ledit élément (23) est un corps absorbant et ladite lame de coupe (65) a une configuration pour découper le corps absorbant.

8. L'organe de coupe (10) selon la revendication 7, l'organe de coupe (10) étant monté sur un appareil (1) pour produire une protection interlabiale présentant le corps absorbant.

9. Une méthode de coupe pour découper un élément (23) en appuyant une lame de coupe (65) contre une feuille continue de matériau de base (130), comprenant les étapes suivantes :
transporter le matériau de base (130) suivant une direction constante ;
gaufrer la feuille continue de matériau de base (130) de façon à ce qu'un taux de gaufrage de portions latérales (130a, 130c) de la feuille continue de matériau de base (130) soit plus élevé qu'un taux de gaufrage d'une portion centrale (130b) dans la direction en largeur de la feuille continue de matériau de base (130) ;
découper l'élément (23) en appuyant ladite lame de coupe (65) contre le matériau de base (130) ;
décharger l'élément (23) qui est découpé par la lame de coupe (65) ; et
diviser un restant de matériau de base (131), lequel est un restant de la feuille continue de matériau de base (130) après que l'élément (23) est découpé, en deux parties (133a, 133c) suivant la direction constante de la feuille continue de matériau de base (130), ladite étape de division du restant de matériau de base (131) en deux parties (133a, 133c) comprenant en outre les étapes suivantes :
former des fentes sous forme de lignes planes de coupe par perforation le long substantiellement d'un centre de la feuille continue de matériau de base (130) dans la direction constante en utilisant une lame (53) pour appuyer contre la feuille continue de matériau de base (130) ; et
décharger les deux parties (133a, 133c) du restant de matériau de base (131) dans des directions qui s'éloignent l'une de l'autre.

10. La méthode de coupe selon la revendication 9 dans laquelle, dans ladite étape de déchargement, chacune des deux parties (133a, 133c) du restant de matériau de base (131) est déchargée dans une direction en épaisseur de l'élément (23) à un angle prédéterminé par rapport à une direction de déchargement de l'élément (23).

11. La méthode de coupe selon la revendication 9 dans laquelle, dans l'étape de déchargement, chacune des deux parties (133a, 133c) du restant de matériau de base (131) est déchargée dans substantiellement la même direction qu'une direction de déchargement de l'élément (23) lorsque vu depuis la direction en largeur de l'élément (23).

12. La méthode de coupe selon une quelconque des revendications 9 à 11, dans laquelle le taux de gaufrage des portions latérales (130a, 130c) de la feuille continue de matériau de base (130) est de 15 % ou plus et le taux de gaufrage de la portion centrale (130b) de celle-ci est de 0,5 % à 10 %.

13. La méthode de coupe selon une quelconque des revendications 9 à 12 dans laquelle, dans ladite étape de coupe, ladite lame de coupe (65) est prévue sur un rouleau (64) et ladite lame de coupe (65) est appuyée contre le matériau de base (130) en faisant tourner ledit rouleau (64) de façon à découper l'élément (23) dans le matériau de base (130).

14. La méthode de coupe selon une quelconque des revendications 9 à 13, la méthode de coupe étant incorporée dans une méthode pour produire une protection interlabiale.
